(19)

Europäisches
Patentamt

European
Patent Office

Office européen
des brevets

(11)  **EP 2 508 598 A1**

(12) **EUROPEAN PATENT APPLICATION**
published in accordance with Art. 153(4) EPC

(43) Date of publication:
**10.10.2012  Bulletin 2012/41**

(21) Application number: **10834526.5**

(22) Date of filing: **26.11.2010**

(51) Int Cl.:
*C12N 9/20* (2006.01)       *C12P 7/62* (2006.01)
*C12N 9/96* (2006.01)       *C12N 11/10* (2006.01)

(86) International application number:
**PCT/JP2010/071095**

(87) International publication number:
**WO 2011/068076 (09.06.2011 Gazette 2011/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **01.12.2009  JP 2009273685**

(71) Applicant: **The Nisshin OilliO Group, Ltd.
Tokyo 104-8285 (JP)**

(72) Inventors:
• **NEGISHI Satoshi
Yokosuka-shi
Kanagawa 239-0832 (JP)**

• **YAMAUCHI Yoshie
Yokosuka-shi
Kanagawa 239-0832 (JP)**
• **NAKAMURA Yosuke
Yokosuka-shi
Kanagawa 239-0832 (JP)**
• **TOYAMA Yuko
Yokosuka-shi
Kanagawa 239-0832 (JP)**

(74) Representative: **Bawden, Peter Charles
Bawden & Associates
4 The Gatehouse, 2 High Street
Harpenden, Herts
AL5 2TH (GB)**

(54)  **LIPASE POWDER PREPARATION AND USE THEREOF**

(57)    The present invention discloses a powdery lipase preparation in which the particles constituting the powdery lipase have 3,000 to 40,000 pores/mm$^2$ on the surface thereof, and each pore has a diameter of 0.5 $\mu$m to 6 $\mu$m. This powdery lipase preparation was able to improve lipase activity without using a soybean powder.

**EP 2 508 598 A1**

**Description**

Technical Field of the Invention

The present invention relates to a powdery lipase preparation which can be preferably used in various esterification reactions or transesterification reactions; and method of transesterification and the like using said powdery lipase preparations.

Background of the Invention

Lipases are widely used in esterification of various carboxylic acids such as fatty acids with alcohols such as monoalcohols and polyalcohols, transesterification between esters of several carboxylic acids, and the like. Among them, the transesterification reaction is an important technology not only as method for modifying animal and vegetable fats and oils but also as method for producing esters of various fatty acids such as sugar esters and sterol esters. When a lipase, which is an enzyme hydrolyzing fats and oils, is used as a catalyst in the above reactions, the transesterification reaction can be conducted under the mild condition, i.e. at room temperature to about 70°C. Therefore, the reactions using a lipase can better inhibit side reactions and reduce energy costs as compared with the conventional chemical reactions. In addition, since a lipase as a catalyst is a natural product, it is highly safe. Further, intended compounds can be effectively produced by using a lipase due to the substrate specificity and positional specificity thereof. However, even if a powdery lipase is used in the transesterification reaction without change, activity thereof does not fully express. Besides, it is difficult to uniformly disperse a basically water-soluble lipase into oil raw materials and to collect such a lipase. Therefore, generally, it is common to immobilize a lipase to some carriers, such as an anion-exchange resin (Patent Literature 1), a phenol adsorption resin (Patent Literature 2), a hydrophobic carrier (Patent Literature 3), a cation-exchange resin (Patent Literature 4) and a chelate resin (Patent Literature 5) and to use it in the reactions such as esterification and transesterification. Further, the method for producing immobilized lipase particles is proposed which comprises the steps of producing an emulsion wherein a water phase dissolving a lipase and a substance which acts as a carrier of a lipase is dispersing into a hydrophobic phase; and removing water from the emulsion to convert the water phase into solid particles thereof covered with the lipase (Patent Literature 6).

As mentioned above, a lipase has been conventionally immobilized and used in the transesterification reaction. However, the immobilized lipase loses an original lipase activity through the immobilization. In addition, when a porous carrier is used, a raw material or a product gets stuck in pores and, as a result, the transesterification rate decreases. Further, in the transesterification reaction wherein the conventional immobilized lipase is used, water which a carrier retains is brought into the reaction system, and therefore, it has been difficult to prevent side reactions such as production of diglycerides and monoglycerides in the transesterification reaction of fats and oils.

In light of the above situations, various technologies using a powdery lipase have been developed. For example, the method of the transesterification reaction is proposed which comprises the steps of dispersing a powdery lipase, in the presence or absence of an inactive organic solvent(s), into a raw material comprising an ester(s) so that 90% or more of the dispersed powdery lipase particles maintains a particle diameter of 1-100 $\mu$ m during the reaction; and transesterifying said mixture (Patent Literature 7). Further, use of an enzymatic powder is also proposed, said enzymatic powder which is obtained by drying an enzyme solution comprising phospholipids and lipid-soluble vitamins (Patent Literature 8).

However, a powdery lipase of which lipase activity is further improved has been desired.

On the other hand, the method for producing an enzyme-immobilized preparation is proposed which comprises the steps of adding a grain powder or a grain powder and sugars to a solution comprising an enzyme(s), and drying the solution comprising an enzyme(s) (Patent Literature 9). The literature discloses that examples of usable enzymes include a lipase, a cellulase, a protease, an amylase and a pectinase, and that the enzyme-immobilized preparation obtained by the above production method can inhibit enzyme deactivation in the presence of a substance reducing an enzymatic activity. However, there is no description on the improvement of an enzymatic activity therein. Further, actually produced examples in the literature are only those in which a defatted soybean powder having less fat content is applied as a cellulase or a protease, and there is no specific description on an example wherein a lipase is used.

Under such circumstances, Patent Literature 10 proposes a powdery lipase preparation which is a granulated material comprising a lipase derived from *Rhizopus oryzae* and/or a lipase derived from *Rhizopus delemar* and a soybean powder having a fat content of 5 mass% or more; and the method for producing the powdery lipase preparation by spray drying. The literature discloses that a powdery lipase preparation of which lipase activity is drastically improved can be obtained.

Patent Literature 1: JP-A 60-98984

(continued)

| Patent Literature 2: | JP-A 61-202688 |
| Patent Literature 3: | JP-A 2-138986 |
| Patent Literature 4: | JP-A 3-61485 |
| Patent Literature 5: | JP-A1-262795 |
| Patent Literature 6: | JP-B 3403202 |
| Patent Literature 7: | JP-B 2668187 |
| Patent Literature 8: | JP-A 2000-106873 |
| Patent Literature 9: | JP-A 11-246893 |
| Patent Literature 10: | JP-A 2008-54448 |

Disclosure of the Invention

The object of the present invention is to provide a powdery lipase preparation of which lipase activity is improved.

The additional object of the present invention is to provide a method of transesterification or a method of esterification in which the powdery lipase preparation is used.

The inventors thoroughly studied physical characteristics of the particles constituting a powdery lipase preparation and the relation thereof with lipase activity, and found that the number of pores existing on the surface of the particles significantly affects lipase activity. The present invention has been completed based on this finding.

Namely, the present invention provides a powdery lipase preparation in which the particles constituting the powdery lipase preparation have 3,000 to 40,000 pores/mm$^2$ on the surface thereof, and each pore has a diameter of 0.5 $\mu$m to 6 $\mu$m.

The present invention also provides a method for producing the powdery lipase preparation which comprises the step of spray drying an aqueous liquid containing a lipase at the blow temperature of 40°C or higher and lower than 70°C.

The present invention further provides a method for producing an esterified product which comprises the step of transesterification or esterification using the powdery lipase preparation.

According to the present invention, it is possible to provide a powdery lipase preparation having a drastically improved enzymatic activity by which the transesterification or esterification reaction can be effectively conducted.

Particularly, according to the present invention, it becomes possible to provide a powdery lipase preparation having a drastically improved enzymatic activity from a lipase itself. Therefore, usage of the powdery lipase preparation in the transesterification or esterification reaction can be decreased, or the reaction time can be drastically shortened. As a result, it is possible to safely and inexpensively produce foods or food additives.

Brief Description of the Drawings

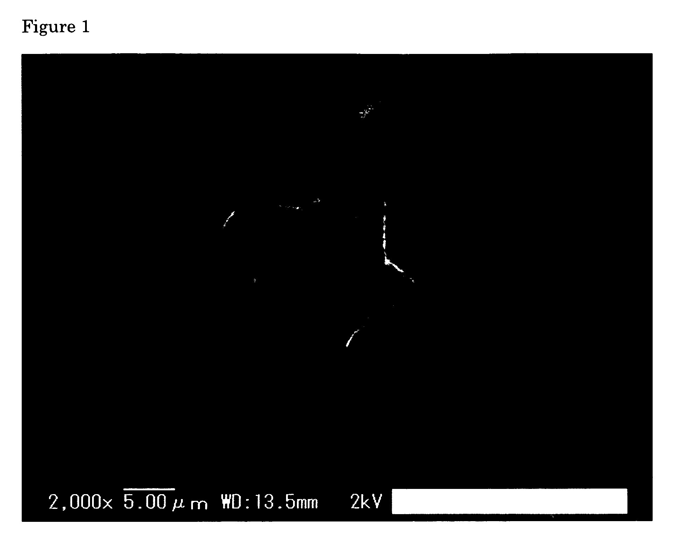

Figure 1 shows a 2000x electron microscope photograph of a lipase particle constituting a powdery lipase preparation obtained by spray drying at the blow temperature of 50°C.

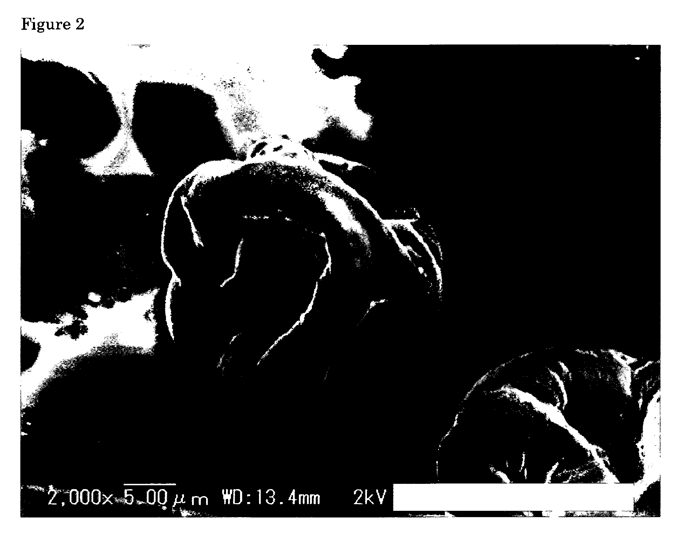

Figure 2 shows a 2000x electron microscope photograph of a lipase particle constituting a powdery lipase preparation obtained by spray drying at the blow temperature of 110°C.

Best Mode for Carrying out the Invention

Various lipases can be used in the present invention. *Rhizopus delemar* and *Rhizopus oryzae* of *Rhizopus* sp. are preferable, and a 1,3-specific lipase is particularly preferable. Among them, *Rhizopus oryzae* is further more preferable.

Examples of these lipases include Picantase R8000 (a product of Robin) and Lipase F-AP15 (a product of Amano Enzyme Inc.). The most preferable lipase is Lipase DF "Amano" 15-K (also referred to as Lipase D) derived from *Rhizopus oryzae* or Lipase D "Amano" Conc. each product of Amano Enzyme Inc. These products are powdery lipases. Meanwhile, Lipase DF "Amano" 15-K was originally referred to as a lipase derived from *Rhizopus delemar*.

A lipase used in the present invention can be those obtained by drying an aqueous solution containing a lipase, such as an aqueous solution containing a medium component of a lipase. However, a lipase not containing them, i.e., a lipase substantially composed of a lipase itself is preferable.

It is preferable to produce the powdery lipase preparation of the present invention by dissolving and/or dispersing a lipase with a grain powder and/or a sugar powder to prepare an aqueous liquid containing a lipase, and spray drying said aqueous liquid.

As for an amount of water in an aqueous liquid containing a lipase, the mass of water is adjusted corresponding to the mass of a lipase. More specifically, the mass of water per the mass of a lipase is preferably 2.0-1,000 times, more preferably 2.0-500 times and most preferably 3.0-100 times.

Examples of the aqueous liquid containing a lipase herein used include a lipase culture from which fungus body is removed, a purified culture, an aqueous solution wherein a lipase obtained from said culture(s) is dissolved or dispersed again, an aqueous solution wherein a marketed powdery lipase is dissolved or dispersed again, and a marketed liquid lipase. Further, the aqueous solutions from which a low-molecular-weight component such as salts is removed are more preferable in order to further improve lipase activity. In addition, the aqueous solutions from which a low-molecular-weight component such as sugars is removed are more preferable in order to further improve powder properties.

The culture of a lipase is conducted by using, as a lipase culture, aqueous solutions containing soybean flour, peptone, corn steep liquor, $K_2HPO_4$, $(NH_4)_2SO_4$, $MgSO_4 \cdot 7H_2O$ or the like. The concentrations thereof are as follows: soybean flour is 0.1-20 mass% and preferably 1.0-10 mass%; peptone is 0.1-30 mass% and preferably 0.5-10 mass%; corn steep liquor is 0.1-30 mass% and preferably 0.5-10 mass%; $K_2HPO_4$ is 0.01-20 mass% and preferably 0.1-5 mass%; $(NH_4)_2SO_4$ is 0.01-20 mass% and preferably 0.05-5 mass%; and $MgSO_4 \cdot 7H_2O$ is 0.01-20 mass% and preferably 0.05-5 mass%. The cultural conditions thereof can be controlled as follows: the cultural temperature is 10-40°C and preferably 20-35°C; the quantity of airflow is 0.1-2.0VVM and preferably 0.1-1.5VVM; the rotation speed for stirring is 100-800rpm and preferably 200-400rpm; pH is 3.0-10.0 and preferably 4.0-9.5.

The separation of a fungus body after the culture of a lipase is preferably conducted by centrifugation, membrane filtration, or the like. The removal of low-molecular-weight components such as salts and sugars can be treated with ultrafiltration membranes. More specifically, after the treatment with ultrafiltration membranes, an aqueous solution containing a lipase is concentrated so as to become 1/2 volume thereof; and then, the same amount of a phosphate buffer as that of the concentrated solution is added thereto. By repeating these procedures 1-5 times, it is possible to obtain an aqueous solution containing a lipase from which a low-molecular-weight component is removed.

The centrifugal force of the centrifugation is preferably controlled to 200-20,000 $\times$ g. Similarly, the pressure of the membrane filtration is preferably controlled to 3.0kg/m$^2$ or less in microfiltration membranes, the filter press and the like. In the case of enzymes in the fungus body, it is preferable to crush cells thereof by a homogenizer, Waring blender, ultrasonic disruption, French press, ball mill or the like, and then to remove cell residues by centrifugation, membrane filtration, or the like. The rotation speed for stirring of the homogenizer is 500-30,000rpm and preferably 1,000-15,000rpm. The rotation speed of Waring blender is 500-10,000rpm and preferably 1,000-5,000rpm. The time for stirring is 0.5-10 minutes and preferably 1-5 minutes. The ultrasonic disruption is conducted in the condition of 1-50 KHz and preferably 10-20 KHz. It is preferable that the ball mill has glass pellets having the diameter of 0.1-0.5mm.

In some stage before the drying process, it is possible to concentrate an aqueous solution containing a lipase. The concentration method is not particularly limited, and examples thereof include an evaporator, a flash evaporator, the concentration by ultrafiltration, the concentration by microfiltration, salting out by inorganic salts, precipitation with solvents, absorption with ion-exchange celluloses and the like, and water absorption with water-absorbing gels. The concentration by ultrafiltration and an evaporator are preferable among them. The module of the concentration by ultrafiltration is as follows: a flat membrane or a hollow fiber membrane each having a fractionated molecular weight of 3,000-100,000 and more preferably 6,000-50,000; and the material thereof is preferably polyacrylonitrile, polysulphone, or the like.

A lipase used in the present invention is preferably those from which fungus body and constituents contained in a lipase culture are removed. This lipase and a grain powder and/or a sugar powder are mixed in water and spray dried. Though the order of mixing is not particularly limited, it is preferable to disperse a grain powder and/or a sugar powder in an aqueous solution in which a lipase is dissolved in water. Preferable examples of a grain powder and a sugar powder include soybean powders such as a whole fat soybean powder and a defatted soybean powder, flour, rice flour and dextrin.

As the method of spray drying an aqueous liquid containing a lipase, for example, there is the method of spray drying with spray dryers such as a countercurrent flow dryer with a nozzle(s), a countercurrent flow dryer with a disk, a concurrent flow dryer with a nozzle(s) and a concurrent flow dryer with a disk. In that case, it is preferable to adjust the temperature of an aqueous liquid containing a lipase and a soybean powder to 20-40°C, and then to conduct spray drying (to spray the liquid in dry atmosphere). It is preferable to spray the liquid at the blow (dry atmosphere) temperature of 40°C or higher and lower than 70°C, more preferably 40°C-65°C, and further more preferably 40°C-60°C. Further, it is also preferable to adjust pH of an aqueous liquid containing a lipase to 7.5-8.5 before drying.

A lipase is vulnerable to temperature, and the reduction in an enzymatic activity thereof can be inhibited by keeping the lipase to a low temperature. However, in the present invention, the blow temperature of 40°C or higher and lower than 70°C is more preferable than the blow temperature of low temperature area, i.e. 16°C-30°C, since the lipase activity becomes higher in the former condition.

In the present invention, a powdery lipase preparation constituted by the particles having 3,000 to 40,000 pores/mm$^2$ on the surface thereof wherein each pore has a diameter of 0.5 $\mu$m to 6 $\mu$m can be obtained by the above method. The particles preferably have 3,000 to 20,000 pores/mm$^2$, and more preferably 3,000 to 10,000 pores/mm$^2$ on the surface thereof and each pore has a diameter of 0.5 $\mu$m to 6 $\mu$m. The number of pores on the surface of the particles can be easily measured by using an electron microscope.

At that time, it is preferable to obtain a powdery lipase preparation having a water content of 10 mass% or less, and particularly preferably 1-8 mass%.

A particle diameter of a powdery lipase preparation of the present invention can be arbitrarily selected, and 90 mass% or more of the powdery lipase preparation preferably has a particle diameter of 1-150 $\mu$m. The average particle diameter thereof is preferably 10-80 $\mu$m. Further, a form of the powdery lipase preparation is preferably spherical.

The particle diameter of a powdery lipase preparation can be measured by using a particle size distribution analyzer (LA-500) of HORIBA, Ltd, for example.

Next, described herein is the method for producing a transesterified product or an esterified product each of which is obtained by conducting transesterification or esterification using a powdery lipase preparation of the present invention.

The transesterification reaction conducted using a powdery lipase preparation of the present invention is a transesterification reaction of esters of fatty acids with one or more kinds selected from esters of fatty acids, fatty acids and alcohols. Examples thereof include transesterification between fats and oils in accordance with the ordinary method, transesterification of fats and oils with esters of fatty acids, and transesterification of alcoholysis or acidolysis.

Further, the esterification reaction conducted by using a powdery lipase preparation of the present invention is an esterification reaction of partial esters of fatty acids with fatty acids, or an esterification reaction of mono- or poly-alcohols with fatty acids. Examples thereof include an esterification reaction of glycerin with fatty acids.

More specifically, as the transesterification reaction between fats and oils, it is possible to transesterify canola oil which is a triglyceride of a long-chain fatty acid and a glycerol trioctanoate which is a triglyceride of a medium-chain fatty acid derived from vegetables, and to produce a triglyceride which comprises long-chain and medium-chain fatty acids.

Further, as the transesterification reaction of fats and oils and fatty acids using acidolysis, it is possible to produce structured fats and oils wherein a 1,3-specific lipase that a lipase has is significantly used. This is the method that a specific fatty acid is left on the second position of glycerin skeleton and fatty acids on the first and third positions are replaced by intended fatty acids. The obtained fats and oils can be used as those for chocolates and those having specific nutritional effects.

The condition of the transesterification reaction or the esterification reaction using a powdery lipase preparation of the present invention is not particularly limited, and each reaction can be conducted by the ordinary method.

Generally, the reaction is conducted under ordinary or reduced pressure with preventing contamination of water that causes hydrolysis. Though the reaction temperature depends on a used raw material such as fats and oils and the freezing point of a mixture of a raw material, it is preferably 20-80°C, and if not limited by the freezing point, it is preferably 40-60°C.

The additive amount of a powdery lipase preparation in a reaction raw material is preferably 0.05-10 mass%, and more preferably 0.05-5 mass%. The most suitable amount is determined in accordance with the reaction temperature, set reaction time, activity of the obtained powdery lipase preparation, and the like. After the reaction completed, a powdery lipase preparation is removed by filtration and centrifugation, and it can be repeatedly used (evaluation of stability) until the activity thereof decreases to the extent that the production of a powdery lipase preparation is impossible.

Accordingly, it is desired that a lipase, which is usually expensive, can give both high activity and high stability in the smallest possible amount thereof to a powdery lipase preparation, and this can be accomplished by using a powdery lipase preparation of the present invention.

Though thus obtained transesterified or esterified product is not particularly limited, it is useful as transesterified or esterified fats and oils used in the food field.

Next, Examples will further illustrate the present invention.

Examples

Example 1

A marketed product of Amano Enzyme Inc., Lipase D "Amano" Conc. Lot. No. LDD0252201 was dissolved in water to prepare 33,600 U/mL of an enzyme solution thereof. Three times amount of a 10% suspension of a deodorized whole fat soybean powder (fat content: 23 mass %; trade name: Alphaplus HS-600, produced by The Nisshin OilliO Group, Ltd.) was added with stirring to the enzyme solution. Then, pH thereof was adjusted to 7.8 with a 0.5N NaOH solution to obtain an aqueous liquid containing a lipase. This aqueous liquid containing a lipase was introduced to a low temperature spray dryer, and spray dried at various blow temperatures.

The activity of each of thus obtained powdery lipase preparations was measured in accordance with the following method.

Measurement method of lipase activity

Each powdery lipase preparation was added to oil in which 1,2,3-trioleoyl glycerol and 1,2,3-trioctanoyl glycerol were mixed in 1:1(w), and reacted at 60°C. 10 μL thereof was taken as a sample over time, diluted with 1.5mL of hexane, and a solution wherein the powdery lipase preparation was filtered was taken as a sample for gas chromatography (GC). The solution was analyzed by GC (column: DB-1ht) and the reaction rate was calculated from the following formula. The GC conditions are: column temperature: 150°C, temperature rising: 15°C/min., and final temperature 370°C.

$$\text{Reaction rate (\%)} = \{\text{C34area}/(\text{C24area} + \text{C34area})\} \times 100$$

wherein C24 is 1,2,3-trioctanoyl glycerol; C34 is 1,2,3-trioctanoyl glycerol wherein one fatty acid is replaced by an oleic acid; and area is each area thereof. Based on the reaction rate of each time, the reaction rate constant k was calculated by an analysis software (origin ver. 6.1).

As for the activity of the powdery lipase preparation, the activity thereof at each blow temperature was represented by the relative activity when defining the activity of the powdery lipase preparation produced at the blow temperature of 110°C as 100.

Table 1 shows results thereof.

Table 1: Blow temperature in the spray drying and relative activity of lipase

| Blow temperature (°C) | 16 | 30 | 50 | 90 | 110 |
|---|---|---|---|---|---|
| Relative activity (%) | 145 | 146 | 166 | 140 | 100 |

From the above results, it is clarified that a powdery lipase having a high relative activity can be obtained when setting the blow temperature to 40°C or higher and lower than 70°C in the spray drying.

Next, in order to examine a cause of producing such a powdery lipase having a high relative activity, the number of pores on the surface of each lipase particles constituting the powdery lipase of Table 1 was checked in accordance with the following method.

<Details on the method of analysis>

The surface of lipase particles constituting the powdery lipase was examined under an electron microscope. The magnification was set to 1500x or 2000x so that one grain of powdery lipase (a lipase particle) randomly selected could fit onto the screen. A part of the examined surface (around the center of the particle) was cut out (the area of 200-600 μm$^2$), and the number of pores having a diameter of 0.5 μm to 6 μm therein was counted visually. Then, the number of pores on the surface/surface area (mm$^2$) was calculated therefrom.

This operation was conducted to 5 samples per each group, and the average value thereof was calculated. Table 2 shows results thereof.

Table 2: Blow temperature and the number of pores on the surface of the obtained powdery lipase

| Blow temperature (°C) | Number of pores on the surface / surface area (mm$^2$) |
|---|---|
| 50°C | 4,044 |
| 60°C | 8,376 |
| 110°C | 1,839 |

From the above results, it is clarified that rather than the difference in the blow temperatures in the pray drying, the number of pores on the surface of the obtained powdery lipase correlates with the relative activity of lipase.

Meanwhile, the amount of water in each of powdery lipases produced at the blow temperatures of 50°C and 110°C was 7.2 mass% and 2.9 mass%, respectively (measured by Karl Fischer's method). The powdery lipase produced at the blow temperature of 50°C was dried to decrease the amount of water thereof, and the powdery lipase produced at the blow temperature of 110°C was moisturized so that the amount of water of each powdery lipase became 5.7 mass%. Then, when checking the relative activity of lipase of each powdery lipase, it was clarified that the number of pores on the surface of the powdery lipase affects the relative activity of lipase more than the amount of water.

**Claims**

1.  A powdery lipase preparation in which the particles constituting the powdery lipase preparation have 3,000 to 40,000 pores/mm$^2$ on the surface thereof, and each pore has a diameter of 0.5 $\mu$m to 6 $\mu$m.

2.  The powdery lipase preparation according to claim 1, wherein the particles have 3,000 to 20,000 pores/mm$^2$ on the surface thereof, and each pore has a diameter of 0.5 $\mu$m to 6 $\mu$m.

3.  The powdery lipase preparation according to claim 1 or 2, wherein the lipase is derived from *Rhizopus oryzae.*

4.  The powdery lipase preparation according to any one of claims 1 to 3, wherein 90 mass% or more of the particles have a particle diameter of 1-150 $\mu$m.

5.  The powdery lipase preparation according to any one of claims 1 to 4 which is used for transesterification or esterification.

6.  The powdery lipase preparation according to any one of claims 1 to 5, which is produced by spray drying at the blow temperature of 40°C or higher and lower than 70°C.

7.  A method for producing the powdery lipase preparation according to any one of claims 1 to 5 which comprises the step of spray drying an aqueous liquid containing a lipase at the blow temperature of 40°C or higher and lower than 70°C.

8.  A method for producing an esterified product which comprises the step of transesterification or esterification using the powdery lipase preparation according to any one of claims 1 to 5.

Figure 1

2,000x 5.00 μm WD:13.5mm    2kV

Figure 2

## INTERNATIONAL SEARCH REPORT

| International application No. |
|---|
| PCT/JP2010/071095 |

**A. CLASSIFICATION OF SUBJECT MATTER**
*C12N9/20*(2006.01)i, *C12P7/62*(2006.01)i, *C12N9/96*(2006.01)n, *C12N11/10*
(2006.01)n

According to International Patent Classification (IPC) or to both national classification and IPC

**B. FIELDS SEARCHED**

Minimum documentation searched (classification system followed by classification symbols)
C12N9/20, C12P7/62, C12N9/96, C12N11/10

Documentation searched other than minimum documentation to the extent that such documents are included in the fields searched

Electronic data base consulted during the international search (name of data base and, where practicable, search terms used)
CA/BIOSIS/MEDLINE/WPIDS(STN), JSTPlus(JDreamII)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2008/114656 A1 (The Nisshin Oillio Group, Ltd.),<br>25 September 2008 (25.09.2008),<br>& US 2010/0112650 A1 & EP 2141229 A1 | 1-5,8<br>6,7 |
| A | JP 2007-68426 A (The Nisshin Oillio Group, Ltd.),<br>22 March 2007 (22.03.2007),<br>(Family: none) | 1-8 |
| A | WO 2005/097985 A1 (The Nisshin Oillio Group, Ltd.),<br>20 October 2005 (20.10.2005),<br>& US 2006/0105438 A1 & EP 1734115 A1 | 1-8 |

☒ Further documents are listed in the continuation of Box C.     ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>09 December, 2010 (09.12.10) | Date of mailing of the international search report<br>21 December, 2010 (21.12.10) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2010/071095

C (Continuation). DOCUMENTS CONSIDERED TO BE RELEVANT

| Category* | Citation of document, with indication, where appropriate, of the relevant passages | Relevant to claim No. |
|---|---|---|
| A | WO 2005/097984 A1 (The Nisshin Oillio Group, Ltd.), 20 October 2005 (20.10.2005), & US 2006/0105935 A1 & US 2009/0104680 A1 & EP 1734114 A1 | 1-8 |
| A | ENGSTROM JD et al., Stable high surface area lactate dehydrogenase particles produced by spray freezing into liquid nitrogen, Eur. J. Pharm. Biopharm., 2007, Vol.65, No.2, pp.163-174 | 1-8 |
| P,A | WO 2010/089967 A1 (Fuji Oil Co., Ltd.), 12 August 2010 (12.08.2010), (Family: none) | 1-8 |

Form PCT/ISA/210 (continuation of second sheet) (July 2009)

## REFERENCES CITED IN THE DESCRIPTION

**Patent documents cited in the description**

- JP 60098984 A
- JP 61202688 A
- JP 2138986 A
- JP 3061485 A
- JP 262795 A

- JP 3403202 B
- JP 2668187 B
- JP 2000106873 A
- JP 11246893 A
- JP 2008054448 A